# EUROPEAN PATENT SPECIFICATION

(11) **EP 1 570 844 B1**
(45) Date of publication and mention of the grant of the patent: **29.11.2006**
(21) Application number: 03777040.1
(22) Date of filing: 01.12.2003
(51) Int. Cl.: A61K 31/445, A61K 31/135, A61P 11/00

(54) **COMPOSITION COMPRISING PSEUDOEPHEDRINE AND DOMPERIDONE FOR PREVENTING SNORING**
ZUSAMMENSETZUNG ENTHALTEND PSEUDOEPHEDRIN UND DOMPERIDONE ZUM VERHINDERN DES SCHNARCHENS
PRODUIT PHARMACEUTIQUE PERMETTANT DE SOULAGER OU DE FAIRE DISPARAITRE LES RONFLEMENTS

(30) Priority: 02.12.2002 CL 27602002
(43) Date of publication of application: 07.09.2005
(73) Proprietor: LARRA N ORREGO, Augusto, Las Condes, Santiago (CL)
(72) Inventor: LARRA N ORREGO, Augusto, Las Condes, Santiago (CL)
(74) Representative: Bublak, Wolfgang
(86) International application number: PCT/IB2003/005652
(87) International publication number: WO 2004/050070

(56) References cited:
- US-A- 6 114 346
- FIREMAN P: "THERAPEUTIC APPROACHES TO ALLERGIC RHINITIS: TREATING THE CHILD" JOURNAL OF ALLERGY AND CLINICAL IMMUNOLOGY, MOSBY - YEARLY BOOK, INC, US, vol. 105, no. 6, 2000, pages S616-S621, XP001097923 ISSN: 0091-6749

## Description

This invention is aimed at severe snorer (kind of snorer who disturbs or does not allow the cohabitation) which is a normal person without the characteristics of the pathologic snore of the sleep apnoea.

The interest for sleep apnoea has increased during the last years since the studies carried out have showed that it is a very common pathology which can be associated to severe cardiovascular disorders in case of no treatment. The access to more reliable diagnostic methods and effective treatments contribute to the advancement of the research and the diagnostic and therapeutic evaluation by specialists.

They don't exist oral treatments which allows to control the loud snore proper to severe snorer, nor prospective studies, by random election and double blind method control experiment, which lasted and demonstrated to be harmless or minimum-collateral effects. Being the severe snorer a normal person with no pathology, invasive treatments should be avoided, such as the treatments currently used for the sleep apnoea, which is a pathologic condition that may associate with cardiovascular alterations.

Up to date a several number of homeopathic drugs do exist, being these drugs based on natural plants and herbs which fluidize secrections mucosae of the palate, in order to facilitate the passage of the air. Furthermore, there are a lot of "spray", nasal drops and local or habitational aromatherapy, nasal lubricators and external correctors to lubricate nasal graves. In the USA more than 50 devices suitable for local placing such as sosmoguard among others have been patented, and there are specially inflatable pillows apt to prevent for sleeping on shoulder, and strips or collars which close the mouth by raising the mandible.

156.080 websites informing about these treatments for snorers were detected through an Internet search, where a wide range of things, from hypnosis to surgery, can be found.

There are various invasive treatments, among which the mostly used are the following:
1. Snoroplasty by injection, which infiltrates alcohol or tetradecyl sodium sulphate directly into palate in order to harden it. Its effects appear after weeks and, according to the authors, the resulting sclerosis is useful.
2. Laser Uvulopalatoplasty, which is currently very popular, including in our country (Chile). Said technique consists in laser ablation of flabby palate and uvula in order to create a larger duct. Without considering its aggressiveness, its results are arguable, according to a remote control of otorrinos (*Arch. Otolaryngol. Head Neck Surg.,* **2001**, 127, 412-417), which throw a 43% of prior satisfaction, that decrease in further controls. In said publication, authors stress that fibrosis and adherences of residuary palate may appear, which causes sleep apnoea in a large number of patients who didn't have this disease before.

The last publication is aimed to nocturnal nasal congestion which might be an important independent factor in usual snore without true apnoea. The authors made carried out polysomnography on 4,916 patients in Wisconsin (USA) and concluded that patients affected by severe chronic nasal congestion had more chances to be affected by usual snores (*Arch. Intern. Med*., **2001,** 161, 1514-1519).

Finally, we have to mention the use of CPAP, a system composed by a pressure oxygen machine for patients with sleep apnoea who need to use it during the whole night. Its commercial value is of 1.000.000 Chile pesos for each case and our Health system can not afford their provision as in other countries.

The use by the inventor, at the beginning of the seventies, of an effective surgery in case of gastro-oesophageal reflux brought to various consequences not related before. According to patients, the day after surgery they could breathe better, asthmatic patients didn't have crisis anymore and obstructive bronchitis disappeared in a lot of babies. That motivated the author to make a study during 15 years; this study was the first work made with strict method and control, internationally recognized. In 1974 relationship between larynx alteration and gastro-oesophageal reflux, which now is well known as "acid laryngitis", appeared. This kind of alteration produces congestion of respiratory mucosa, being the hoarseness its main symptom. Furthermore, it's very important to stress that in most cases this alteration is not accompanied by (oesophageal reflux) symptoms, or these symptoms are very weak. Its incidence in normal population is very high. The concomitance with snore disappearance in patient after surgery, told by relatives, motivated the inventor to find a medical treatment for alteration of respiratory way which exists in snorers who are nor affected by an anatomical obstructive alteration (such as adenoid, pronounced deviations of nasal wall, etc.). Association between larynx hurt and congestion brought to possibility of using a decongestant, which was added with an acid protective substance against gastro-oesophageal reflux that increases gastric and oesophageal mortality. Nevertheless, they do not add substances which decrease or delete acid secretion, because this kind of substances interfere with the latter drug absorbability; furthermore, the most of the patients do not relate discomforts due to presence of gastric ebbs.

Pseudoephedrine has 71.000 search results in Internet and domperidone has 9.080). In Chile, pseudoephedrine was included among drugs with no medical prescription from May 1, 2000. In this sense, the Head of Public Health Institute (ISP), Mr. Gonzalo Navarrete, mentioned at this time that: "it is senseless to restrict it as it is not a dangerous substance". On the other hand, the current Head of the ISP, Mrs. Jeannette Vega pointed out on August 17, 2002 that "drugs with no prescription do not show a risk of improper use, abuse or addiction. The eventual side effects showed are not severe and are reversible once the treatment is stopped. For that reason, these are safe and effective medicines to be consumed by the population.

We can optionally use, as an average range, a combination of pseudoephedrine sulphate (60 mg) and pseudoephedrine hydrochloride (120 mg).

PVA103 was invented this way; PVA103 is made by pseudoephedrine (60 mg) and domperidone (10 mg) each capsule. Both drugs have been broad studied.

The combination of pseudoephedrine with domperidone is new. This combination hasn't been showed in any country all over the world, nor its use for snorer. The 25% of population may be scheduled as normal snorer, moderate or severe. This alteration generates life together problems and possibility of lessening this situation using a simple drug is very important.

In Chile, the pseudoephedrine is used in various preparations, being associated with ascorbic acid within 4 preparations, with brompheniramine within 1 preparation, with caffeine within 2 preparations, with cetirizine within 2 preparations, with chlorpheniramine within 45 preparations, with codeine within 15 preparations, with eucalyptus within 1 preparation, with loratidine within 10 preparations, with noscapine within 3 preparations, with paracetamol within 31 preparations, with propyphenazone within 1 preparation, with retinol within 1 preparation, with toluol within 1 preparation and with tripolidine within 2 preparations

PVA103 was tested by volunteers, who took a capsule 30 minutes after dinner and were allowed to drink a moderate quantity of alcohol or two glasses of wine. If after 4 hours volunteer do snore again, he is supplied with another dose. During these tests no discomforts were detected and results are very good because of snore disappearance.

Beyond the clear benefit for patients, this drug is a very low-cost one, which allows a massive and beneficial use due to the new use of two known compounds, which supplied in the mentioned dose constitutes a new compound which resolves a technical problem with no equivalent solution.

## Claims

1. Composition comprising pseudoephedrine and domperidone.

2. Pharmaceutical composition comprising pseudoephedrine and domperidone.

3. Use of pseudoephidrine and domperidone for preparing a pharmaceutical composition for lightening or eliminating shore in a moderate or severe snorer.

4. Use according to claim 3, wherein said pharmaceutical composition is suitable for oral administration and is in capsule form.

5. Use according to claim 3, wherein said pharmaceutical composition comprises 60 mg pseudoephidirine per dose and 10 mg domperidone per dose.

6. Use according to claim 3, wherein said pharmaceutical composition is administered once a day, preferable 30 minutes after dinner.

## Patentansprüche

1. Zusammensetzung, umfassend Pseudoephedrin und Domperidon.

2. Arzneimittel, umfassend Pseudoephedrin und Domperidon.

3. Verwendung von Pseudoephedrin und Domperidon zur Herstellung eines Arzneimittels zum Erleichtern oder Eliminieren des Schnarchens bei einem mäßigen oder starken Schnarcher.

4. Verwendung nach Anspruch 3, wobei das Arzneimittel zur oralen Verabreichung geeignet ist und in Kapselform vorliegt.

5. Verwendung nach Anspruch 3, wobei das Arzneimittel 60 mg Pseudoephedrin pro Dosis und 10 mg Domperidon pro Dosis umfasst.

6. Verwendung nach Anspruch 3, wobei das Arzneimittel einmal täglich, vorzugsweise 30 Minuten nach dem Abendessen verabreicht wird.

## Revendications

1. Composition comprenant de la pseudoéphédrine et de la dompéridone.

2. Composition pharmaceutique comprenant de la pseudoéphédrine et de la dompéridone.

3. Utilisation de la pseudoéphédrine et de la dompéridone pour préparer une composition pharmaceutique pour soulager ou éliminer le ronflement chez un ronfleur modéré ou grave.

4. Utilisation selon la revendication 3, où ladite composition pharmaceutique est appropriée à une administration orale et est sous forme de gélule.

5. Utilisation selon la revendication 3, où ladite composition pharmaceutique comprend 60 mg de pseudoéphédrine par dose et 10 mg de dompéridone par dose.

6. Utilisation selon la revendication 3, où ladite composition pharmaceutique est administrée une fois par jour, de préférence 30 minutes après dîner.
